# EUROPEAN PATENT APPLICATION

(11) **EP 4 450 620 A1**
(43) Date of publication of application: **23.10.2024**
(21) Application number: 23746005.0
(22) Date of filing: 13.01.2023
(51) Int. Cl.: C12N 9/12, C12P 19/34

(54) **TERMINAL TRANSFERASE VARIANT FOR CONTROLLABLE SYNTHESIS OF SINGLE-STRANDED DNA AND USE THEREOF**

(30) Priority: 28.01.2022 CN 202210107337
(71) Applicant: Tianjin Institute of Industrial Biotechnology, Chinese Academy of Sciences, Tianjin 300308 (CN)
(72) Inventor: JIANG, Huifeng, Tianjin 300308 (CN); LU, Xiaoyun, Tianjin 300308 (CN); LI, Congyu, Tianjin 300308 (CN); CHENG, Jian, Tianjin 300308 (CN); LU, Lina, Tianjin 300308 (CN); LOU, Qianqian, Tianjin 300308 (CN)
(74) Representative: Gille Hrabal Partnerschaftsgesellschaft mbB Patentanwälte
(86) International application number: PCT/CN2023/072086
(87) International publication number: WO 2023/143123

(57) **Abstract**

The present disclosure discloses a plurality of terminal deoxynucleotidyl transferases (TdTs) and variants thereof for *de novo* synthesis of polynucleotides having controlled sequences and efficiently controllable synthesis of nucleic acid molecules without depending on a template. It is found that some amino acid residues in the TdT catalytic domain can be specifically modified to improve the ability of such modified TdTs to synthesize polynucleotides.

## Description

The present application claims priority to the prior Application with the patent application No. 2022101073377 entitled "TERMINAL TRANSFERASE VARIANTS FOR CONTROLLABLY SYNTHESIZING SINGLE-STRANDED DNA AND USE THEREOF" and filed with China National Intellectual Property Administration on Jan. 28, 2022. which is incorporated herein by reference in its entirety.

### TECHNICAL FIELD

The present disclosure relates to a terminal deoxynucleotidyl transferase and use thereof, and particularly to a terminal transferase variant for the controlled synthesis of single-stranded DNA and use thereof, belonging to the technical field of biology.

### BACKGROUND

DNA is a carrier of life information, and obtaining DNA is the first step in research, modification, and creation of life. DNA synthesis is one of the core enabling technologies in synthetic biology. Genomic DNA can be synthesized on a large scale, which is helpful to improve our understanding, prediction and regulation of organisms and provide us with the ability to design and modify cellular functions and even reconstruct artificial life.

A number of researchers have attempted to synthesize DNA by chemical and enzymatic methods from the 1950s to the present. The chemical synthesis technique was the first to be successful. After years of optimization and improvement, DNA chemical synthesis has undergone a revolutionary development from column synthesis to microchip synthesis and has been widely used in the market. However, for the chemical method, on one hand, coupling efficiency and side reactions limit the synthetic length of the oligonucleotide within 300 nt, and it is difficult to achieve the gene length in kb. Thus, the splicing of oligonucleotide fragments is required by biological assembly techniques to obtain longer fragments until DNA having the gene, chromosome or genome length is obtained. On the other hand, the reaction steps are complicated, the time consumed for a single cycle is long (6-8 min), the consumption of chemical reagents is high, the cost is high, and a large amount of toxic and flammable organic reagents are used, resulting in great pollution.

Therefore, the synthesis of DNA by biological enzymes has been the focus of research. However, conventional DNA polymerases have template-dependency and cannot synthesize DNA *de novo.* The search for template-independent DNA polymerases becomes a primary task for enzymatic synthesis. In 1962, Bollum first discovered terminal deoxynucleotidyl transferase (TdT), and proposed that TdT could be used for single-stranded oligonucleotide synthesis. At present, the enzymatic oligonucleotide synthesis adopts a nucleotide with a modification group at position 3 as a substrate. After the reaction of this substrate with the phosphate group at position 5 of the primer strand, a further incorporation of the nucleotide into the oligonucleotide strand is blocked since the modification group is unable to react with the phosphate group at position 5 of the next nucleotide. The incorporation of only one nucleotide with a modification group can be catalyzed per enzymatic reaction, and then the modification group is removed by chemical or biological methods to revert to OH at the 3' end for the next synthetic cycle. However, the wild-type protein has low catalytic efficiency and the accuracy cannot be achieved in this process, so there is an urgent need to find an enzyme with high catalytic activity and relative stability.

### SUMMARY

To solve the above problems, the inventors of the present application found that some amino acid residues of TdT catalytic domain can be specifically modified to improve the ability of TdT variants to synthesize polynucleotides by studying various terminal deoxynucleotidyl transferases (TdTs) and variants thereof for *de novo* synthesis of polynucleotides having controlled sequences and efficiently controllable synthesis of nucleic acid molecules without depending on a template.

The present disclosure adopts the following technical schemes:
The present disclosure provides a terminal deoxynucleotidyl transferase (TdT) variant, which is any one or a combination of site mutants or terminal variants of ZaTdT SEQ ID NO.1 derived from *Zonotrichia albicollis:*
The present disclosure provides a plurality of terminal deoxynucleotidyl transferase (TdT) site mutants having a function of catalyzing deoxyribonucleotides with O-amino modification groups at the 3' end, the site mutants comprising at least one mutation of a residue in at least one position selected from Y178, F186, I210, I228, V302, D324, R353, D324, T330, G331, G332, F333, R335, K337, I329, I339, G340, H341, I343, R435, G452, W453, T454, G455, S456, R457, N477, and H478 or a functionally equivalent residue, the position indicated being determined by alignment with SEQ ID NO.1.

According to the present disclosure, "a functionally equivalent residue" and "functionally equivalent residues" mean residues in a sequence of TdT which is homologous to the sequence set forth in SEQ ID NO.1 and has the same functional effect. The functionally equivalent residues are identified using sequence alignments, for example, Mutalin online alignment software (http://multalin.toulouse.inra.fr/multalin/multalin.html; 1988, Nucl. Acids Res., 16(22), 10881-10890). In addition, the functionally equivalent residues can be determined by three-dimensional structural alignment for proteins. After alignment, the functionally equivalent residues are at homologous positions on the different sequences considered. Sequence alignments and identification of functionally equivalent residues may be performed between any TdT and its natural variants, including interspecies variants.

According to the present disclosure, an amino acid sequence of the site mutant comprises at least one mutation of a residue in at least one position selected from Y178, F186, I210, I228, V302, T330, R335, K337, I339, G340, R435, T454, G455, S456, R457, N477, and H478, or a functionally equivalent residue.

According to the present disclosure, the amino acid sequence of the site mutant comprises at least one mutation of a residue in at least one position selected from Y178A, F186R, I210L, I228L, V302G, T330A, R335A, R335L, K337A, K337G, K337L, I339V, G340G, G340L, R435L, T454A, G455V, S456V, R457G, N477L, and H478G, or a functionally equivalent residue.

According to the present disclosure, the amino acid sequence of the site mutant comprises mutations of amino acid residues at any two of sites Y178, F186, I210, I228, V302, D324, R353, D324, T330, G331, G332, F333, R335, K337, I329, I339, G340, H341, I343, R435, G452, W453, T454, G455, S456, R457, N477, and H478.

According to the present disclosure, the amino acid sequence of the site mutant comprises mutations of amino acid residues at any two of sites Y178, F186, I210, I228, V302, T330, R335, K337, I339, G340, R435, T454, G455, S456, R457, N477, and H478.

According to the present disclosure, the amino acid sequence of the site mutant comprises mutations of amino acid residues at two sites of Y178, F186, I210, I228, V302, T330, R335, K337, I339, G340, R435, T454, G455, S456, R457, N477, and H478.

According to the present disclosure, the amino acid sequence of the site mutant comprises mutations at the following two sites: Y178/F186, I210/I228, R335/K337, I339/G340, and S456/R457. According to the present disclosure, the amino acid sequence of the site mutant comprises mutations at the following two sites: Y178A/F186R, I210L/I228L, R335A/K337A, R335L/K337G, R335L/K337L, R335L/K337V, I339V/G340V, and S456V/R457L.

According to the present disclosure, the terminal variant of TdT is selected from any one or a combination of the following:
According to the present disclosure, the terminal variant of TdT includes variants that add or delete some amino acids to or from SEQ ID NO.1 or the above site mutants or functionally equivalent sequences, which still have the function of synthesizing nucleic acids using 3'-O-modified nucleotides. Preferably, the sequence of the variant is set forth in SEQ ID NO.2.

According to the present disclosure, the terminal variant of TdT includes changes that do not affect the activity of proteins, for example the inclusion of tag sequences, such as MBP tag, HIS tag, GST tag, etc., at the N-terminus and/or C-terminus of SEQ ID NO.1 or the above site mutants or functionally equivalent sequences. Preferably, the sequence of the variant is set forth in SEQ ID NO.3 or SEQ ID NO.4.

According to the present disclosure, variants of TdT have substitutions or combinations of substitutions as described above and have at least 80% identity to SEQ ID NO.1 or a functionally equivalent sequence, preferably at least 85%, 90%, 95%, 96%, 97%, 98%, or 99% identity to SEQ ID NO.1 or a functionally equivalent sequence.

According to the present disclosure, all variants of TdT as disclosed above are capable of both synthesizing nucleic acid fragments in the absence of a template and incorporating modified nucleotides into nucleic acid fragments. Advantageously, the variant has an increased ability to incorporate the modified nucleotides, preferably 3'-O-modified nucleotides, into the nucleic acid fragments compared to TdT set forth in SEQ ID NO.1.

In some embodiments as described above, the efficiency of the variant of TdT in incorporating 3'-O-modified nucleoside triphosphates is at least 110% of that of the wild-type TdT with a sequence SEQ ID NO.1. In other embodiments, the efficiency of the variant of TdT in incorporating 3'-O-modified nucleoside triphosphates is at least 150% of that of the wild-type TdT with a sequence SEQ ID NO.1. In other embodiments, the efficiency of the variant of TdT in incorporating 3'-O-modified nucleoside triphosphates is at least 200% of that of the wild-type TdT with a sequence SEQ ID NO.1. According to the present disclosure, the variants of TdT are capable of incorporating modified nucleotides, preferably 3'-O-modified nucleotides, and more preferably 3'-O-blocked nucleotides. In the context of the present disclosure, the expression "modified nucleotide" refers to a molecule containing a nucleoside (i.e. a base attached to a deoxyribose or ribose molecule) binding to three phosphate groups, the nucleoside having at least one additional group at one of its ends (2', 3', 5', or a base). The additional group blocks further addition of nucleotides by preventing formation of any phosphodiester bond (3'-O-modification, 2' or 2'-O-modification), or by sterically preventing polymerase attachment to any nucleic acid fragment comprising such modified nucleotides (5' or base modification) at the 3' end. In addition, the additional group advantageously has reversible properties, allowing this group to be removed by a specific cleaving reaction.

In one specific embodiment, the variant further comprises at least one mutation of residues in at least a semi-conserved region of X1X2X3X4RX5GX6NX7X8X9DX10 from position 330 to position 343 of the sequence, wherein
X1 represents a residue selected from A, I, L, V, P, Q, T, K, and E,
X2 represents a residue selected from G, L, V, F, Y, C, H, R, and D,
X3 represents a residue selected from A, I, L, V, P, Q, T, K, and E,
X4 represents a residue selected from G, L, V, F, Y, C, H, R, and D,
X5 represents a residue selected from A, I, L, V, P, Q, T, K, and E,
X6 represents a residue selected from G, L, V, F, Y, C, H, R, and D,
X7 represents a residue selected from A, I, L, V, P, Q, T, K, and E,
X8 represents a residue selected from G, L, V, F, Y, C, H, R, and D,
X9 represents a residue selected from A, I, L, V, P, Q, T, K, and E, and
X10 represents a residue selected from G, L, V, F, Y, C, H, R, and D.

In another specific embodiment, the variant further comprises at least one mutation of residues in at least a semi-conserved region of ALLX1X2X3X4X5X6QFG from position 449 to position 460 of the sequence, wherein
X1 represents a residue selected from A, I, L, V, P, Q, T, K, and E;
X2 represents a residue selected from G, L, V, F, Y, C, H, R, and D;
X3 represents a residue selected from A, I, L, V, P, Q, T, K, and E;
X4 represents a residue selected from G, L, V, F, Y, C, H, R, and D;
X5 represents a residue selected from A, I, L, V, P, Q, T, K, and E; and
X6 represents a residue selected from G, L, V, F, Y, C, H, R, and D.

According to the present disclosure, the variant comprises at least one combination of substitutions selected from combinations of substitutions disclosed in Table 1, or functionally equivalent residues; preferably, the variant comprises at least one combination of substitutions selected from combinations of substitutions disclosed in Table 3, or functionally equivalent residues.

Another object of the present disclosure is to provide a plurality of terminal deoxynucleotidyl transferase variants having nucleotides that catalyze non-natural 3'-OH modifications. The terminal transferase variant has no naturally resolved crystal structure, and the three-dimensional structure is obtained by homologous modeling with Alpha fold2.

The present disclosure also provides a nucleic acid molecule encoding the variant of TdT as defined above.

The present disclosure also provides an expression vector comprising the nucleic acid molecule described herein.

The present disclosure also provides a host cell, wherein the host cell comprises the nucleic acid molecule or the expression vector described herein.

The present disclosure further provides a method for producing a variant of TdT, wherein the host cell as defined above is cultured under a culture condition allowing the expression of the nucleic acid encoding the variant, and wherein the variant is optionally recovered.

The present disclosure also relates to use of the variant of TdT for the synthesis of a nucleic acid molecule with one or more 3'-O-modified nucleotides in the absence of a template.

In some embodiments, the method comprises the steps of: (a) providing an initiating fragment comprising an oligonucleotide having a free 3'-hydroxyl; (b) reacting the TdT variant of the present disclosure with the initiating fragment or an extended initiating fragment in the presence of a 3'-O-reversibly blocked nucleoside under an enzymatic extension condition.

In some embodiments, the method further comprises the steps of: (c) deblocking the extended initiating fragment to form an extended initiator fragment having a free 3'-hydroxyl, and (d) repeating steps (b) and (c) until a nucleic acid molecule having a predetermined sequence is synthesized.

A further object of the present disclosure is to provide a method for synthesizing a nucleic acid molecule in the absence of a template, comprising a step of contacting a nucleic acid primer with at least one nucleotide, preferably at least one 3'-O-modified nucleotide, and a variant of TdT according to the present disclosure.

The present disclosure also provides a kit, enzyme or nucleotide composition for performing a nucleotide incorporation reaction, wherein the kit, enzyme or nucleotide composition comprises a variant of TdT according to the present disclosure.

According to the present disclosure, the kit, enzyme or nucleotide composition further comprises one or more nucleotides (preferably one or more 3'-O-modified nucleotides) or a nucleoside molecule. According to the present disclosure, the modified nucleotide is fluorescently labeled for detection. Further, the kit, enzyme or nucleotide composition described herein further comprises optionally at least one DNA template molecule or nucleic acid primer.

Preferably, the kit, enzyme or nucleotide composition for performing a nucleotide incorporation reaction comprises 1) a variant of TdT provided according to the present disclosure; 2) one or more nucleotides (preferably one or more 3'-O-modified nucleotides); and 3) optionally at least one nucleic acid primer.

### Beneficial Effects of Present Disclosure:

1) The present disclosure provides a plurality of terminal deoxynucleotidyl transferase mutants, which can synthesize nucleic acid molecules efficiently and controllably without depending on a template, and surprisingly discovers that the efficiency of the plurality of mutants is significantly higher than that of wild-type terminal deoxynucleotidyl transferase before modification.
2) The plurality of terminal deoxynucleotidyl transferase mutants provided by the present disclosure can be applied to controllable and accurate synthesis of single-stranded DNA.

### BRIEF DESCRIPTION OF THE DRAWINGS

FIG. 1 is a schematic diagram showing the catalytic reaction of TdT and a variant thereof.
FIG. 2 is a graph showing the relative activity of some TdT variants in catalyzing a reversible termination modification group at the 3 end.
FIG. 3 is a graph showing the denaturing PAGE result of 10 cycles of nucleotide synthesis using the TdT variants.

### Description of the Disclosure

The DNA polymerase family is divided into 7 families according to their sequence homology and crystal structure. Among them, the polymerases of PolX family represent a wide range of polymerases from replicative polymerases to terminal transferases. The polymerases from the PolX family are present in a very wide range of eukaryotic organisms. The polymerases from the PolX family are involved in a wide variety of biological processes, in particular DNA damage repair mechanisms or error correction mechanisms. The PolX family is subdivided into polymerase β (Pol β), µ (Pol µ), λ (Pol λ), IV from yeast (Pol IV), and terminal deoxynucleotidyl transferase (TdT). TdT is naturally involved in DNA repair and maintenance mechanisms. In particular, TdT has the unique ability to preserve nucleotide polymerization activity even in the absence of a template strand. Under certain conditions, TdT is capable of extending a DNA fragment by several hundred nucleotides using natural nucleotides in the absence of any complementary strand. However, wild-type TdT is completely ineffective for incorporation of sugar-modified nucleotides.

Therefore, an object of the present disclosure is to provide variants of TdT with targeted mutations that allow the incorporation of modified nucleotides into a nucleic acid fragment by the variants of TdT during synthesis of the nucleotide fragment. More particularly, the present inventors have identified specific amino acid residues that can be advantageously substituted, alone or in combination, to improve the ability of the enzyme to synthesize nucleic acid fragments of different lengths with a predetermined sequence, including the ability to synthesize nucleic acid fragments of different lengths and having a predetermined sequence by using modified nucleotides.

### Definitions

As used herein, the terms "mutant" and "variant" are used interchangeably to refer to a polypeptide derived from SEQ ID NO.1 and comprising a modification or alteration, i.e., a substitution, insertion and/or deletion, at one or more (e.g., several) positions and having both polymerase activity in the absence of a template and the ability to incorporate one or more modified terminator nucleotides. Variants can be obtained by various techniques known in the art. In particular, examples of techniques for altering a DNA sequence encoding a wild-type protein include, but are not limited to, site-directed mutagenesis, random mutagenesis, and synthetic oligonucleotide construction. Mutagenic activity includes the deletion, insertion or substitution of one or several amino acids in the sequence of a protein or the polymerase in terms of the present disclosure. The targeted amino acids may be accompanied or distributed throughout the sequence of the polymerase. For example, a specific motif or structural feature may be targeted.

The term "modification" or "alternation" as used herein with respect to the position of an amino acid means that the amino acid at a specific position is modified compared to the amino acid of the wild-type protein.

"Substitution" means that one amino acid residue is replaced by another amino acid residue. Preferably, the term "substitution" refers to the replacement of one amino acid residue by another amino acid residue selected from: 20 standard naturally occurring amino acid residues, rare naturally occurring amino acid residues (e.g., hydroxyproline, hydroxylysine, allohydroxylysine, 6-*N-*methyllysine, *N*-ethylglycine, *N*-methylglycine, *N*-ethylasparagine, allo-isoleucine, *N-*methylisoleucine, *N*-methylvaline, pyroglutamine, aminobutyric acid, ornithine, norleucine, and norvaline), and non-naturally occurring amino acid residues that are typically synthetically produced (e.g., cyclohexylalanine). Preferably, the term "substitution" refers to the replacement of one amino acid residue by another amino acid residue selected from the 20 standard naturally occurring amino acid residues. The symbol "+" indicates a combination of substitutions.

Herein, amino acids are represented by their one-letter or three-letter codes according to the following nomenclature: A: alanine (Ala); C: cysteine (Cys); D: aspartic acid (Asp); E: glutamic acid (Glu); F: phenylalanine (Phe); G: glycine (Gly); H: histidine (His); I: isoleucine (Ile); K: lysine (Lys); L: leucine (Leu); M: methionine (Met); N: asparagine (Asn); P: proline (Pro); Q: glutamine (Gln); R: arginine (Arg); S: serine (Ser); T: threonine (Thr); V: valine (Val); W: tryptophan (Trp), and Y: tyrosine (Tyr).

In this document, the following terms are used to indicate a substitution: L238A shows that the amino acid residue (leucine, L) at position 238 of the parent sequence is changed to alanine (A). A132V/I/M shows that the amino acid residue (alanine, A) at position 132 of the parent sequence is substituted with one of the following amino acids: valine (V), isoleucine (I), or methionine (M). The substitution may be a conservative or non-conservative substitution. Examples of conservative substitutions are within the group of basic amino acids (arginine, lysine and histidine), acidic amino acids (glutamic acid and aspartic acid), polar amino acids (glutamine, asparagine and threonine), hydrophobic amino acids (methionine, leucine, isoleucine, cysteine and valine), aromatic amino acids (phenylalanine, tryptophan and tyrosine) and small amino acids (glycine, alanine and serine).

As used herein, the term "sequence identity" or "identity" refers to the number (or fraction expressed in %) of matches (identical amino acid residues) between two polypeptide sequences. The sequence identity is determined by comparing sequences when aligned so as to maximize overlap and identity while minimizing sequence gaps. In particular, the sequence identity can be determined using any of a variety of mathematical global or local alignment algorithms, depending on the length of the two sequences. Sequences of similar length are preferably aligned using a global alignment algorithm (e.g., Needleman and Wunsch algorithm; Needleman and Wunsch,1970) that optimally aligns the sequences over their entire length, while sequences of substantially different lengths are preferably aligned using a local alignment algorithm (e.g., Smith and Waterman algorithm (Smith and Waterman, 1981) or Altschul algorithm (Altschul et al., 1997; Altschul et al., 2005)). Alignment for purposes of determining percent amino acid sequence identity can be achieved in various ways that are within the skill in the art, for example, using publicly available computer software available on internet websites such as http://blast.ncbi.nlm.nih.gov/ or http://www.ebi.ac.uk/Tools/emboss/. Those skilled in the art can determine appropriate parameters for measuring alignment, including any algorithms required to achieve maximum alignment of the full length of the aligned sequences. For purposes herein, % amino acid sequence identity values refer to values generated using the pairwise sequence alignment program EMBOSS Needle, which creates an optimal global alignment of two sequences using the Needleman-Wunsch algorithm, wherein all search parameters are set to default values, i.e. Scoring matrix = BLOSUM62, Gap open = 10, Gap extend = 0.5, End gap penalty = false, End gap open = 10, and End gap extend = 0.5.

As used herein, the terms "peptide", "polypeptide", "protein", and "enzyme" refer to a chain of amino acids linked by peptide bonds, regardless of the number of amino acids forming the chain.

Unless otherwise indicated, the positions disclosed in the present application are numbered by reference to the amino acid sequence set forth in SEQ ID NO. 1.

### Variants of TdT

The present disclosure provides variants of TdT enzymes that can be used to synthesize polynucleotides having predetermined sequences, such as DNA or RNA, in the absence of a template strand. The TdT variants of the present disclosure allow for the use of modified nucleotides, and in particular 3'-O-modified nucleotides, in enzyme-mediated polynucleotide synthesis methods.

In the context of the present disclosure, "modified terminal deoxynucleotidyl transferase", "modified TdT", "variant of terminal deoxynucleotidyl transferase", and "variant of TdT" refer to one that shares at least 25% identity to the amino acid sequence of TdT. Preferably, the variant of TdT shares at least 40% identity to SEQ ID NO. 1.

TdT is known to be composed of different domains from the N-terminus to the C-terminus, corresponding to the nuclear localization signal domain (NLS), BRCT-like domain and catalytic domain (C-TdT), respectively.

The variants of the present disclosure are described by their mutations at specific residues, the positions of which are determined by alignment with or reference to the enzyme sequence SEQ ID NO.1. However, in the context of the present disclosure, any variant having a sequence functionally equivalent to SEQ ID NO. 1 is also part of the present disclosure. In the same way, any variant bearing the same mutation at functionally equivalent residues is also part of the present disclosure.

In the context of the present disclosure, "a functionally equivalent sequence" refers to the sequence of TdT homologous to SEQ ID NO.1. "Functionally equivalent residues" means residues in the sequence of TdT which is homologous to the sequence set forth in SEQ ID NO.1 and has the same functional effect. The functionally equivalent residues are identified using sequence alignments, for example, Mutalin online alignment software (http://multalin.toulouse.inra.fr/multalin/multalin.html; 1988, Nucl. Acids Res., 16(22), 10881-10890). After alignment, the functionally equivalent residues are at homologous positions on the different sequences considered. Sequence alignments and identification of functionally equivalent residues may be performed between any TdT and its natural variants, including interspecies variants.

TdT may be found in many other organisms or microorganisms. All of these TdT are good candidates for carrying out the present disclosure. In particular, modifications that alter a particular TdT sequence to confer the polymerase with an increased ability to incorporate modified nucleotides, may target any other TdT sequence. Therefore, mutations or combinations described herein by reference to SEQ ID NO. 1, and more particularly to those corresponding to amino acid residues 330 to 343 and 449 to 460 of SEQ ID NO.1, can be transposed to any other TdT sequence.

More particularly, the present disclosure provides variants of TdT with at least one substitution or a combination of substitutions as listed in Table 1. The variants of the present disclosure comprise at least amino acid substitutions listed in the left column and referred to as "variable mutations", or functionally equivalent residues, and optionally one or a combination of two substitutions listed in the right column and referred to as "optional constant mutations", or functionally equivalent sequences.

According to the present disclosure, variants of TdT have substitutions or combinations of substitutions as described above and have at least 80% identity to SEQ ID NO.1 or a functionally equivalent sequence, preferably at least 85%, 90%, 95%, 96%, 97%, 98%, or 99% identity to SEQ ID NO.1 or a functionally equivalent sequence.

According to the present disclosure, all variants of TdT as disclosed above are capable of both synthesizing nucleic acid fragments in the absence of a template and incorporating modified nucleotides into nucleic acid fragments. Advantageously, the variant has an increased ability to incorporate the modified nucleotides, preferably 3'-O-modified nucleotides, into the nucleic acid fragments compared to TdT set forth in SEQ ID NO.1.

In some embodiments as described above, the efficiency of the variant of TdT in incorporating 3'-O-modified nucleoside triphosphates is at least 110% of that of the wild-type TdT with a sequence SEQ ID NO.1. In other embodiments, the efficiency of the variant of TdT in incorporating 3'-O-modified nucleoside triphosphates is at least 150% of that of the wild-type TdT with a sequence SEQ ID NO.1. In other embodiments, the efficiency of the variant of TdT in incorporating 3'-O-modified nucleoside triphosphates is at least 200% of that of the wild-type TdT with a sequence SEQ ID NO.1.

### Additional Modifications

In embodiments, the variants of TdT further comprise any type of tagging peptide in their N-terminus, C-terminus, or both termini, such as a His-tag sequence. The tagging peptide can be used for purification, identification, increasing expression, secretability or increasing catalytic activity. It will be understood that such different tags are extensively described in the literature and thus all tags known to a skilled person are encompassed in the present disclosure.

The variants of the present disclosure can further comprise one or more exogenous or heterologous features at the N- and/or C-terminus regions of the protein for use, e.g., in the purification of the recombinant polymerase.

The variants of the present disclosure can further comprise substitutions of residues between positions 330 to 343 and between positions 449 to 460, or functionally equivalent residues, wherein the positions are numbered by reference to the amino acid sequence set forth in SEQ ID NO.1. In another particular embodiment, the variant of TdT comprises at least a substitution of the amino acid sequence SEQ ID NO.1, or a functionally equivalent sequence.

### Modified Nucleotides

According to the present disclosure, the variants of TdT are capable of incorporating modified nucleotides, preferably 3'-O-modified nucleotides, and more preferably 3'-O-blocked nucleotides. In the context of the present disclosure, the expression "modified nucleotide" refers to a molecule containing a nucleoside (i.e. a base attached to a deoxyribose or ribose molecule) binding to three phosphate groups, the nucleoside having at least one additional group at one of its ends (2', 3', 5', or a base). The additional group blocks further addition of nucleotides by preventing formation of any phosphodiester bond (3'-O-modification, 2' or 2'-O-modification), or by sterically preventing polymerase attachment to any nucleic acid fragment comprising such modified nucleotides (5' or base modification) at the 3' end. In addition, the additional group advantageously has reversible properties, allowing this group to be removed by a specific cleaving reaction.

As examples of deoxyribose-containing nucleotides, the nucleotides or nucleoside triphosphates include deoxyadenosine triphosphate (dTAP), deoxyguanosine triphosphate (dGTP), deoxycytidine triphosphate (dCTP), or deoxythymidine triphosphate (dTTP). Adenosine triphosphate (ATP), guanosine triphosphate (GTP), cytidine triphosphate (CTP), or uridine triphosphate (UTP) are further examples of ribose-containing nucleoside triphosphates. Other types of nucleosides, such as naturally occurring modified nucleosides and artificial nucleosides, can bind to three phosphates to form nucleoside triphosphates. In a particular embodiment, the modified nucleotide is a 3'-O-blocked nucleotide comprising a group reversibly attached to the 3' end of a nucleoside triphosphate to prevent further nucleotide addition. The group can have diverse chemical natures, such as azidomethyl, aminoxy, and allyl.

In some embodiments, the modified nucleotide includes a modified nucleotide or nucleoside molecule comprising a purine base or pyrimidine base and a ribose or deoxyribose sugar moiety having a removable 3'-OH blocking group covalently attached thereto such that the 3' carbon atom has attached a group of reversible structure (-O-Z),
wherein -Z is any of -C(R')₂-O-R", -C(R')₂-N(R")₂, -C(R')₂-N(H)R", -C(R')₂-S-R" and -C(R')₂-F, wherein each R" is or is part of a removable protecting group; each R' is independently a hydrogen atom, alkyl, substituted alkyl, arylalkyl, alkenyl, alkynyl, aryl, heteroaryl, heterocyclic, acyl, cyano, alkoxy, aryloxy, heteroaryloxy or an amido group, or a detectable label attached by a linking group; provided that in some embodiments, such substituents have up to 10 carbon atoms and/or up to 5 oxygen or nitrogen heteroatoms; or (R')₂ represents an alkylidene group of formula =C(R‴)₂, wherein each R‴ may be the same or different and is selected from the group comprising hydrogen atoms, halogen atoms and alkyl groups, provided that in some embodiments, the alkyl group of each R‴ has from 1 to 3 carbon atoms; and wherein the molecules can be reacted to produce an intermediate in which each R" is exchanged for H, or where Z is -(R')₂-F, F is exchanged for OH, SH or NH₂, preferably for OH, which intermediate dissociates under aqueous conditions to provide a molecule having a free 3'-OH; provided that when Z is -C(R')₂-S-R", both R' groups are not H. In certain embodiments, R' of the modified nucleotide or nucleoside is alkyl or substituted alkyl, provided that such alkyl or substituted alkyl has from 1 to 10 carbon atoms and from 0 to 4 oxygen or nitrogen heteroatoms. In certain embodiments, -Z of the modified nucleotide or nucleoside is of the formula - C(R')₂-N₃. In certain embodiments, Z is azidomethyl.

In some embodiments, Z is a cleavable organic moiety with or without heteroatoms having a molecular weight of 200 or less. In other embodiments, Z is a cleavable organic moiety with or without heteroatoms having a molecular weight of 100 or less. In other embodiments, Z is a cleavable organic moiety with or without heteroatoms having a molecular weight of 50 or less.

In a further particular embodiment, a "3'-O-modified nucleotide" refers to a nucleoside triphosphate bearing a 3'-O-methyl, 3'-azido, 3'-O-azidomethyl, 3'-O-amino, 3'-aminoxy, or 3'-O-allyl group at the 3' end. In further embodiments, the 3'-blocked nucleoside triphosphate is blocked by a 3'-O-azidomethyl, 3'-aminoxy, or 3'-O-allyl group. In other embodiments, a "3'-O-modified nucleotide" refers to a nucleoside triphosphate bearing esters, ethers, carbonitriles, phosphates, carbonates, carbamates, hydroxylamine, borates, nitrates, sugars, phosphoramide, phosphoramidates, phenylsulfenates, sulfates, sulfones, or amino acids at the 3' end. In some embodiments, the aforementioned 3'-O-blocking group has a molecular weight of 100 or less.

In other embodiments, the 3'-O-blocking group of the present disclosure includes methyl, 3'-O-(2-nitrobenzyl), allyl, amine, azidomethyl, *tert*-butoxyethoxy, or propargyl.

In a further particular embodiment, a "3'-O-modified nucleotide" refers to a nucleoside triphosphate having a terminator effector modification group, such as those described in WO2016034807.

Interestingly, the variants of the present disclosure exhibit an increased affinity for modified nucleotides, as compared to wild-type TdT, thereby showing an increased ability to incorporate such modified nucleotides in a nucleic acid sequence during nucleic acid synthesis. More particularly, the variants of the present disclosure are able to use and efficiently incorporate 3'-O-modified nucleotides (and more particularly, 3'-O-blocked nucleotides) in nucleic acid sequence, which is not possible with wild-type mouse TdT (see Knapp et al., Chem. Eur. J., 2011, 17:2903).

According to a particular aspect, the present disclosure relates to variants of TdT capable of working with modified nucleotides in a nucleic acid enzymatic synthesis process, particularly with 3'-O-modified nucleotides (e.g., 3'-O-blocked nucleotide), and having the ability to produce nucleic acid molecules of long length or derivatives thereof.

### Enzymatic Synthesis of Nucleic Acids

An object of the present disclosure is to provide variants of TdT which may be used in nucleic acid synthesis, such as described in Ybert et al., WO2015/159023; Jensen et al., Biochemistry, 57:1821-1832 (2018); and Hiatt et al., U.S. Pat. No. 5808045. More particularly, an object of the present disclosure is to provide variants of TdT suitable for adding modified nucleotides to an initiating nucleic acid strand. The blocking group can then be removed to allow the addition of a new modified nucleotide.

According to the present disclosure, it is possible to implement successive cycles comprising additions and deprotections by use of a variant of the present disclosure. This method will therefore allow the controlled extension of an initiating nucleic acid strand into a defined sequence by multiple cycles of addition of a reversibly modified nucleotide and further removal of the blocking group. The present disclosure contemplates the use of modified TdTs according to the present disclosure in any enzymatic nucleic acid synthesis method.

A further object of the present disclosure is to provide a method for synthesizing a nucleic acid molecule in the absence of a template, comprising a step of contacting a nucleic acid primer with both at least one nucleotide, preferably at least one 3'-O-modified nucleotide, and a variant of the present disclosure.

The present disclosure contemplates the concept of enzymatic nucleic acid synthesis method. In such method, nucleic acid molecules are *de novo* synthesized in the absence of any template strand. Therefore, ordered sequences of nucleotides are coupled to initiating nucleic acid fragments by means of the variant of the present disclosure. It will be understood that quantitative coupling and more generally high coupling efficiency of each nucleotide to the growing nucleic acid strand is of great importance. It will also be understood that non-terminator nucleotides, such as natural nucleotides or permanently labeled nucleotides, will not allow any control over the sequence synthesized and will result in, for example, uncontrolled and undesired poly-additions.

According to a particular embodiment, the enzymatic nucleic acid synthesis method comprises:
a. providing a nucleic acid molecule linked to a solid support; and
b. reacting a previous nucleic acid molecule with a reversible terminator-modified nucleotide and a variant of TdT according to the present disclosure;

According to another particular embodiment, the enzymatic nucleic acid method comprises:
a. providing a nucleic acid molecule linked to a solid support;
b. adding a reversibly modified nucleotide and a variant of TdT according to the present disclosure;
c. firstly removing one or more reagents from the solid support;
d. reacting a reversible portion of the reversibly modified nucleotide to deprotect the reversibly modified nucleotide for further subsequent extension;
e. secondly removing one or more reagents from the solid support; and
f. optionally and finally cleaving the nucleic acid molecule from the solid support.

According to another particular embodiment, the enzymatic nucleic acid method comprises a cycle subdivided in the following manner:
a. a stage, referred to as the first stage, of extending Xi nucleotides to one end of the fragments, X being possible between 1 and 5, preferably between 1 and 3, i being the number of cycles, making it possible to obtain fragments comprising n + Xi nucleotides, and comprising the following stages:
   - a first attachment stage of attaching a first end of initial nucleic acid fragments comprising n nucleotides or of nucleic acid fragments in the extension process to a first support,
   - a stage of adding reagents required for the variant of TdT,
   - a stage of adding Xi nucleotides to the second end of the nucleic acid fragments by the variant of TdT, X being between 1 and 5, preferably between 1 and 3, i being the number of cycles,
   - an optional stage of removing the undesired reagents from the reaction medium,
   - a stage of detaching the fragments comprising n + Xi nucleotides from the first support, and
   - a first transfer stage of transferring the fragments comprising n + Xi nucleotides;
b. a stage, referred to as the second stage, of purifying the fragments with a correct sequence comprising n + Xi nucleotides, comprising the following successive stages:
   - a second attachment stage of attaching the fragments comprising n + Xi nucleotides to a second support, the attachment being performed by bearing the end of the Xi nucleotides added during the first stage,
   - a stage of removing fragments not attached to the second support,
   - a stage of separating the fragments comprising n + Xi nucleotides from the second support, and
   - an optional stage of removing undesired residual reagents from the reaction medium;
c. a stage, referred to as the third stage, of optionally amplifying, preferably enzymatically amplifying, such as via PCR, fragments with a correct sequence comprising n + Xi nucleotides, comprising the following successive stages:
   - a stage of adding reagents required for the amplification,
   - a stage (optionally consisting of sub-stages that make the process possible) of multiplying the fragments comprising n + Xi nucleotides by a multiplication factor Yi, i being the number of cycles, Y being possible between 1 and 4 × 10¹⁰, preferably between 1 and 1 × 10⁹, and
   - a stage of transferring the fragments comprising n + Xi nucleotides.

Each cycle is carried out in a reaction medium compatible with the enzymatic addition and the enzymatic amplification, such as an aqueous medium. The synthesis method further comprises a stage of final amplification by a multiplication factor Yf at the end of all i extension cycles.

In some embodiments, the method for synthesizing a polynucleotide comprises the steps of: (a) providing an initiating fragment having a free 3'-hydroxyl; (b) reacting the initiating fragment or an extended intermediate having a free 3'-hydroxyl with a variant of TdT of the present disclosure under an extension condition in the presence of a 3'-O-blocked nucleoside triphosphate to produce a 3'-O-blocked extended intermediate; (c) deblocking the extended intermediate to produce an extended intermediate having a free 3'-hydroxyl; and (d) repeating steps (b) and (c) until the polynucleotide is synthesized.

In some embodiments, the method for synthesizing a polynucleotide comprises the steps of: (a) providing an initiating fragment attached to a solid support, the initiator being an oligonucleotide having a free 3'-hydroxyl; (b) reacting the initiating fragment or an extended intermediate having a free 3'-hydroxyl with a variant of TdT of the present disclosure under an extension condition in the presence of a 3'-O-blocked nucleoside triphosphate to produce a 3'-O-blocked extended intermediate; (c) washing the solid support to remove unincorporated 3'-O-blocked nucleoside triphosphates; (d) deblocking the extended intermediate by exposing the solid support to a deblocking agent to produce an extended intermediate having a free 3'-hydroxyl; and (e) repeating steps (b) and (d) until the polynucleotide is synthesized. The method may comprise a further step of cleaving the completed polynucleotide from the solid support.

In some embodiments, for the TdT-catalyzed addition reaction of step (b), the enzymatic conditions may contain from about 0.20 µM to about 200 µM of the nucleotide having a removable blocking moiety that protects the 3'-hydroxyl and from about 0.20 µM to 200 µM of the free and unmodified 3'-hydroxyls derived from the initiating substrate. In some embodiments, the reaction buffer contains about 10 mM to about 500 mM potassium cacodylate buffer (pH between 6.5 and 7.5) and about 0.01 mM to about 10 mM divalent cation (e.g., CoC1₂ or MnC1₂). Other buffer compositions and components may be suitable for particularly desired embodiments of the present disclosure.

In the context of the present disclosure, the expression "cleaving reaction" refers to any action of substance or physical conditions, which is able to cleave the additional group on reversibly modified nucleotides as previously described. Those skilled in the art will be able to determine a cleaving reaction for any previously listed group.

In one embodiment, the cleaving agent is a chemical cleaving agent. In an alternative embodiment, the cleaving agent is an enzymatic cleaving agent.

It will be understood by those skilled in the art that the selection of the cleaving agent is dependent on the type of 3'-nucleotide blocking group used. For example, tris(2-carboxyethyl)phosphine (TCEP) can be used to cleave a 3'-O-azidomethyl group, a palladium complex can be used to cleave a 3'-O-allyl group, or sodium nitrite can be used to cleave a 3'-O-amino group. In particular embodiments, the cleaving reaction comprises: TCEP, a palladium complex or sodium nitrite.

In particular embodiments, the cleaving reaction is performed in the presence of additional components such as denaturants (e.g., urea, guanidinium hydrochloride, formamide, or betaine). In further embodiments, the cleaving reaction is performed with one or more buffers. It will be understood by those skilled in the art that the selection of the buffer is dependent on the exact mechanism of the reaction.

The present disclosure relates to variants of TdT with the ability to incorporate modified nucleotides in a quantitative way. A "quantitative way" or "quantitative reaction" means a reaction that goes to completion, i.e. in which reactants are totally converted into the product. The polymerase that incorporates reversibly modified nucleotides in a quantitative way is a polymerase capable of extending each nucleic acid fragment with all available nucleotides, leading to the conversion of all the initiating fragments of length n to the fragments of length n+1.

As used herein, an "initiating fragment" refers to a short oligonucleotide sequence having a free 3' end, which can be further extended. In one embodiment, the initiating fragment is a DNA initiating fragment. In an alternative embodiment, the initiating fragment is an RNA initiating fragment.

In one embodiment, the initiating fragment has between 3 and 100 nucleotides, in particular between 3 and 20 nucleotides.

In one embodiment, the initiating fragment is single-stranded. In an alternative embodiment, the initiating fragment is double-stranded.

In one embodiment, the initiating fragment is immobilized on a solid support. The initiating fragment can be attached to the solid support by a variety of methods, resulting in a fragment that is stable under a variety of enzymatic or synthetic reaction conditions to which the fragment will be subjected. In one embodiment, the initiating fragment is immobilized on a solid support via a reversible interacting moiety, such as a chemically-cleavable linker, an antibody/immunogenic epitope, a biotin/biotin-binding protein, or glutathione-GST tag. In a further embodiment, the initiating fragment is immobilized on a solid support via a chemically-cleavable linker, such as a disulfide, allyl, or azide-masked hemiaminal ether linker.

In the initiating fragment, the immobilized part contains at least one restriction site. The use of restriction enzymes and restriction sites in selectively hydrolyzing nucleic acid strands at a specific site is described in the literature. Any skilled person will be able to select an appropriate restriction enzyme that will match the initiating fragment cleaving site sequence.

In an alternative embodiment, the initiating fragment contains at least one uridine. Treatment with uracil-DNA glycosylase (UDG) generates an abasic site. Treatment on an appropriate substrate with an apurinic/apyrimidinic (AP) site endonuclease will extract the nucleic acid strand.

### Nucleic Acid Molecules

A further object of the present disclosure is to provide a nucleic acid molecule encoding the variants of the present disclosure. As used herein, a "nucleic acid molecule" refers to a polymer of nucleosides. In one embodiment, the nucleic acid is DNA. In an alternative embodiment, the nucleic acid is RNA. In an alternative embodiment, the nucleic acid is XNA.

It will be understood by those skilled in the art that each of the nucleic acid molecules previously listed may bear modifications on the bases of the nucleotides that make up the polymeric molecule. Such modifications may be natural modifications, such as epigenetic modifications, or non-natural modifications, such as labels.

In one embodiment, the nucleic acid molecule is DNA, RNA or XNA bearing naturally occurring epigenetic modifications such as methylation, hydroxymethylation, formylation, or 5-carboxylation. In one embodiment, the nucleic acid molecule is DNA, RNA or XNA bearing non-naturally occurring modifications such as fluorescent tags, fluorescent labels, or interacting groups.

In one embodiment, the nucleic acid molecule is a polymer molecule having a length of more than 50, 100, 200, 300, 400, 500, 600, 700, 800, 900, 1000, 2000, 3000, 4000, 5000, 6000, 7000, 8000, 9000, 10,000, 15,000, 20,000, 30,000, 40,000, 50,000 or 100,000 nucleotides.

### Use

Described herein is the use of variants of TdT for nucleic acid synthesis, oligonucleotide synthesis, probe synthesis, tagging, nucleic acid amplification, aptamers, therapeutic nucleic acid molecules, drug target discovery and validation, disease diagnosis, metabolic engineering, data storage, crop improvement, library design, sequencing pools, nucleic acid labeling or attachment, or any other use that is involving nucleic acid molecules.

### Production of Variants of TdT

Variants of the present disclosure may be produced by mutating known reference or wild-type TdT-coding polynucleotides, then expressing them using conventional molecular biology techniques. For example, the mouse TdT gene may be assembled from synthetic fragments using conventional molecular biology techniques, e.g. using protocols described by Stemmer et al., Gene, 164: 49-53 (1995); Kodumal et al., Proc. Natl. Acad. Sci., 101: 15573-15578 (2004), or the like; or it may be directly cloned from mouse cells using protocols described by Boule et al., Mol. Biotechnology, 10: 199-208 (1998), or Bentolila et al., EMBO J., 14: 4221-4229 (1995), or the like.

For example, an isolated TdT gene may be inserted into an expression vector, such as pET32 (NOvagen) to give a vector pCTdT, and then the vector pCTdT may be used to prepare and express TdT variant proteins using conventional protocols. Vectors with the correct sequence may be transformed in *E. coli* producer strains.

Transformed strains are cultured using conventional techniques to pellets from which TdT proteins are extracted. For example, the pellets as previously prepared are thawed in a water bath at 30 °C to 37 °C. Once fully thawed, the pellets are resuspended in lysis buffer composed of 50 mM Tris-HCl (Sigma) with a pH of 7.5, 150 mM NaCl (Sigma), 0.5 mM mercaptoethanol (Sigma), 5% glycerol (Sigma), 20 mM imidazole (Sigma) and 1 tablet of 100 mL of protease cocktail inhibitor (Thermofisher). Careful resuspension is carried out in order to avoid premature lysis and remaining of aggregates. Resuspended cells are lysed through several cycles of French press, until full color homogeneity is obtained. A typical pressure used is 14,000 psi. Lysate is then centrifuged for 1 h to 1 h 30 min at 10,000 rpm. Centrifugate is passed through a 0.2 µm filter to remove any debris before column purification.

TdT proteins may be purified from the centrifugate in a one-step affinity procedure. For example, Ni-NTA affinity column (GE Healthcare) is used to bind to the polymerases. Initially, the column has been washed and equilibrated with 15 column volumes of 50 mM Tris-HCl (Sigma) with pH 7.5, 150 mM NaCl (Sigma) and 20 mM imidazole (Sigma). The polymerases are bound to the column after equilibration. Then a washing buffer, composed of 50 mM Tris-HCl (Sigma) with a pH of 7.5, 500 mM NaCl (Sigma) and 20 mM imidazole (Sigma), is applied to the column for 15 column volumes. After washing, the polymerases are eluted with 50 mM Tris-HCl (Sigma) with a pH of 7.5, 500 mM NaCl (Sigma) and 0.5 M imidazole (Sigma). Fractions corresponding to the highest concentration of polymerases of interest are collected and combined in a single sample. The combined fractions are dialyzed against a dialysis buffer (20 mM Tris-HCl, pH 6.8, 200 mM NaCl, 50 mM MgOAc, 100 mM [NH₄]₂SO₄). The dialysate is subsequently concentrated by means of a concentration filter (Amicon Ultra-30, Merk Millipore). The concentrated enzyme is distributed in small aliquots, 50% glycerol is added finally, and those aliquots are then frozen at -20 °C and stored for a long term. 5 µL of various fractions of the purified enzymes are analyzed in SDS-PAGE gels.

### Kit, Enzyme and Nucleotide composition

A particular aspect of the present disclosure relates to use of a composition and a kit comprising a variant of TdT according to the present disclosure or any particular aspect of the present disclosure, and optionally having any combination of one or more components selected from the following: an initiating fragment, one or more reversible terminator nucleotides, additional enzymes, and reagents used in a cleaving reaction. The kit may be used in a method for enzymatic nucleic acid synthesis.

The present disclosure encompasses a composition of matter comprising TdT variants according to the present disclosure or any particular aspect of the present disclosure, wherein reversibly modified nucleotides are mixed with an appropriate buffer at a ratio.

### DETAILED DESCRIPTION

The experimental methods used in the following examples are conventional methods unless otherwise stated.

The materials, reagents and the like used in the following examples are commercially available unless otherwise stated.

The present disclosure is further illustrated in the following examples, without limiting the scope of the present disclosure. The details of some molecular cloning methods vary according to the reagent, enzyme or kit suppliers, and should be performed according to the product instructions, which will not be described in detail in the examples. The various detailed expression conditions, culture conditions, reaction conditions and detection conditions described in the examples are merely exemplary, and the present disclosure is not limited thereto. All conditions that can achieve the corresponding effects are included.

### Example 1. Species Source of TdT Proteins

The species source and amino acid sequence adopted by the present disclosure are as follows: SEQ ID NO.1 (*Zonotrichia albicollis*, ZaTdT).

The enzymes and genes used in this example were synthesized by GENEWIZ, constructed into a pET-28a vector, and located between enzyme cutting sites NdeI and XhoI. The purified proteins were expressed in *E. coli.* For the construction, expression and purification of the ZaTdT protein, the PET-28a vector was taken as an illustrative vector, and the host bacteria was *E. coli.* The present disclosure includes the above examples, but is not limited thereto, and all vectors and host bacteria capable of successfully expressing and purifying the ZaTdT protein should be included.

The present disclosure also truncated some amino acids at the N-terminus of SEQ ID NO.1, and the sequence was set forth in SEQ ID NO.2 (ZaTdT-JD); the present disclosure also added some amino acids at the N-terminus of SEQ ID NO. 1 or SEQ ID NO.2, and the sequence was set forth in SEQ ID NO.3 (MBP-ZaTdT) or SEQ ID NO.4 (MBP-ZaTdT-JD).

### Example 2. Obtainment of Highly Active Variants

According to the present disclosure, a three-dimensional protein structure of ZaTdT from *Zonotrichia albicollis* was obtained by performing homologous modeling, and the residues in the active center within 6A were analyzed. Based on the experience of the inventors, the amino acids at positions 330 to 343 affected the recognition of entry and exit by substrates, the amino acids at positions 449 to 460 affected the incorporation efficiency of newly added bases, and the amino acids at positions 19 to 37 affected the binding conformation of guide strands. The amino groups in these three regions in the ZaTdT enzyme acted together to catalyze the guide strands and newly incorporate the bases, and their single-site mutations or combined mutations interacted with each other and influenced each other. These single-site or multi-site saturation mutations were combined with means such as high-throughput screening and the like to obtained variants with high catalytic efficiency and substrate specificity. The variants included in the present disclosure are shown in the table below.

**Table 1. Mutants contained in the present disclosure**

| No. | Combinations of mutations |
|---|---|
| S1 | Y178A |
| S2 | Y178I |
| S3 | Y178L |
| S4 | Y178V |
| S5 | Y178P |
| S6 | Y178Q |
| S7 | Y178T |
| S8 | Y178K |
| S9 | Y178E |
| S10 | F186G |
| S11 | F186L |
| S12 | F186V |
| S13 | F186Y |
| S14 | F186C |
| S15 | F186H |
| S16 | F186R |
| S17 | F186D |
| S18 | Y178A-F186G |
| S19 | Y178A-F186R |
| S20 | Y178L-F186G |
| S21 | Y178L-F186R |
| S22 | I210A |
| S23 | I210L |
| S24 | I210V |
| S25 | I210G |
| S26 | I210H |
| S27 | I210K |
| S28 | I210P |
| S29 | I210D |
| S30 | I210Y |
| S31 | I228A |
| S32 | I228L |
| S33 | I228G |
| S34 | I228H |
| S35 | I228K |
| S36 | I228P |
| S37 | I228D |
| S38 | I228Y |
| S39 | I210A-I228L |
| S40 | I210L-I228L |
| S41 | V302A |
| S42 | V302L |
| S43 | V302G |
| S44 | D324G |
| S45 | D324L |
| S46 | D324V |
| S47 | D324F |
| S48 | D324Y |
| S49 | D324C |
| S50 | D324H |
| S51 | D324R |
| S52 | R353G |
| S53 | R353L |
| S54 | R353V |
| S55 | R353F |
| S56 | R353Y |
| S57 | R353C |
| S58 | R353H |
| S59 | R353D |
| S60 | D324G-R353G |
| S61 | D324G-R353L |
| S62 | D324F-R353G |
| S63 | D324Y-R353D |
| S64 | T330A |
| S65 | T330L |
| S66 | T330A-G331V |
| S67 | T330L-G331V |
| S68 | T330K-G331V |
| S69 | T330R-G331H |
| S70 | G332A-F333G |
| S71 | G332L-F333G |
| S72 | G332A-F333V |
| S73 | G332L-F333V |
| S74 | F333G |
| S75 | F333L |
| S76 | R335A |
| S77 | R335A-K337G |
| S78 | R335A-K337A |
| S79 | R335A-K337L |
| S80 | R335A-K337V |
| S81 | R335A-K337F |
| S82 | R335A-K337Y |
| S83 | R335A-K337C |
| S84 | R335A-K337C |
| S85 | R335A-K337H |
| S86 | R335A-K337R |
| S87 | R335A-K337D |
| S88 | R335I |
| S89 | R335I-K337G |
| S90 | R335I-K337L |
| S91 | R335I-K337V |
| S92 | R335I-K337F |
| S93 | R335I-K337Y |
| S94 | R335I-K337C |
| S95 | R335I-K337H |
| S96 | R335I-K337R |
| S97 | R335I-K337D |
| S98 | R335L |
| S99 | R335L-K337A |
| S100 | R335L-K337G |
| S101 | R335L-K337L |
| S102 | R335L-K337V |
| S103 | R335L-K337F |
| S104 | R335L-K337Y |
| S105 | R335L-K337C |
| S106 | R335L-K337H |
| S107 | R335L-K337R |
| S108 | R335L-K337D |
| S109 | R335V |
| S110 | R335V-K337G |
| S111 | R335V-K337L |
| S112 | R335V-K337V |
| S113 | R335V-K337F |
| S114 | R335V-K337Y |
| S115 | R335V-K337C |
| S116 | R335V-K337H |
| S117 | R335V-K337R |
| S118 | R335V-K337D |
| S119 | R335P |
| S120 | R335P-K337G |
| S121 | R335P-K337L |
| S122 | R335P-K337V |
| S123 | R335P-K337F |
| S124 | R335P-K337Y |
| S125 | R335P-K337C |
| S126 | R335P-K337H |
| S127 | R335P-K337R |
| S128 | R335P-K337D |
| S129 | R335Q |
| S130 | R335Q-K337G |
| S131 | R335Q-K337L |
| S132 | R335Q-K337V |
| S133 | R335Q-K337F |
| S134 | R335Q-K337Y |
| S135 | R335Q-K337C |
| S136 | R335Q-K337H |
| S137 | R335Q-K337R |
| S138 | R335Q-K337D |
| S139 | R335T |
| S140 | R335T-K337G |
| S141 | R335T-K337L |
| S142 | R335T-K337V |
| S143 | R335T-K337F |
| S144 | R335T-K337Y |
| S145 | R335T-K337C |
| S146 | R335T-K337H |
| S147 | R335T-K337R |
| S148 | R335T-K337D |
| S149 | R335K |
| S150 | R335K-K337G |
| S151 | R335K-K337L |
| S152 | R335K-K337V |
| S153 | R335K-K337F |
| S154 | R335K-K337Y |
| S155 | R335K-K337C |
| S156 | R335K-K337H |
| S157 | R335K-K337R |
| S158 | R335K-K337D |
| S159 | R335E |
| S160 | R335E-K337G |
| S161 | R335E-K337L |
| S162 | R335E-K337V |
| S163 | R335E-K337F |
| S164 | R335E-K337Y |
| S165 | R335E-K337C |
| S166 | R335E-K337H |
| S167 | R335E-K337R |
| S168 | R335E-K337D |
| S169 | K337G |
| S170 | K337L |
| S171 | K337V |
| S172 | K337F |
| S173 | K337Y |
| S174 | K337C |
| S175 | K337H |
| S176 | K337R |
| S177 | K337E |
| S178 | K337C |
| S179 | K337D |
| S180 | I329G |
| S181 | I329L |
| S182 | I329V |
| S183 | I329F |
| S184 | I329Y |
| S185 | I329C |
| S186 | I329H |
| S187 | I329R |
| S188 | I329D |
| S189 | I339A |
| S190 | I339L |
| S191 | I339A-G340L |
| S192 | I339L-G340V |
| S193 | I339V-G340L |
| S194 | I339V-G340V |
| S195 | I339P-G340L |
| S196 | G340L |
| S197 | H341A |
| S198 | H341L |
| S199 | H341A-I343G |
| S200 | H341A-I343L |
| S201 | H341L-I343G |
| S202 | H341L-I343L |
| S203 | H341L-I343V |
| S204 | H341L-I343R |
| S205 | H341V-I343G |
| S206 | H341V-I343V |
| S207 | H341V-I343L |
| S208 | I343G |
| S209 | R435L |
| S210 | R435G |
| S211 | R435A |
| S212 | G452A-W453G |
| S213 | G452A-W453L |
| S214 | G452A-W453V |
| S215 | G452A-W453K |
| S216 | G452L-W453G |
| S217 | G452L-W453L |
| S218 | G452L-W453V |
| S219 | G452L-W453P |
| S220 | G452V-W453G |
| S221 | G452V-W453L |
| S222 | G452V-W453V |
| S223 | G452V-W453K |
| S224 | W453G |
| S225 | W453L |
| S226 | W453V |
| S227 | T454A |
| S228 | T454A-G455L |
| S229 | T454A-G455V |
| S230 | T454A-G455F |
| S231 | T454L-G455L |
| S232 | T454L-G455V |
| S233 | T454L-G455K |
| S234 | T454V-G455L |
| S235 | T454V-G455V |
| S236 | T454V-G455F |
| S237 | G455V |
| S238 | G455L |
| S239 | S456A |
| S240 | S456L |
| S241 | S456V |
| S242 | S456A-R457G |
| S243 | S456A-R457L |
| S244 | S456A-R457V |
| S245 | S456A-R457P |
| S246 | S456L-R457G |
| S247 | S456L-R457L |
| S248 | S456L-R457V |
| S249 | S456L-R457K |
| S250 | S456V-R457G |
| S251 | S456V-R457L |
| S252 | S456V-R457V |
| S253 | S456V-R457P |
| S254 | R457G |
| S255 | R457L |
| S256 | R457V |
| S257 | N477A |
| S258 | N477I |
| S259 | N477L |
| S260 | N477G |
| S261 | N477K |
| S262 | N477R |
| S263 | N477D |
| S264 | N477Y |
| S265 | H478G |
| S266 | H478L |
| S267 | H478V |
| S268 | H478F |
| S269 | H478Y |
| S270 | H478C |
| S271 | H478D |
| S272 | H478R |
| S273 | N477L-H478G |
| S274 | N477R-H478G |
| S275 | N477K-H478G |
| S276 | N477L-H478F |
| S277 | N477L-H478C |

### Example 3. Obtainment of ZaTdT Variant Proteins

For the *in vitro* detection of the ZaTdT variant enzyme activity, the enzyme was exogenously expressed and purified in *E. coli.* The host bacterium described in the examples is *E*. *coli* BL21 (DE3), but the host bacterium is not limited thereto, and all hosts that can be used for expressing proteins are included. The methods for protein expression and purification refer to patent No. 201911034444.6.

### Example 4. Functional Verification

According to the present disclosure, the substrates as used in the examples were all based on deoxyribonucleotides (dNTPs), and the 3'-OH end was changed into a reversible terminating group, including adenine deoxyribonucleotides, guanine deoxyribonucleotides, cytosine deoxyribonucleotides, and thymine deoxyribonucleotides. Preferably, the 3' end modification group is an oxyamino group.

### 1. Schematic diagram

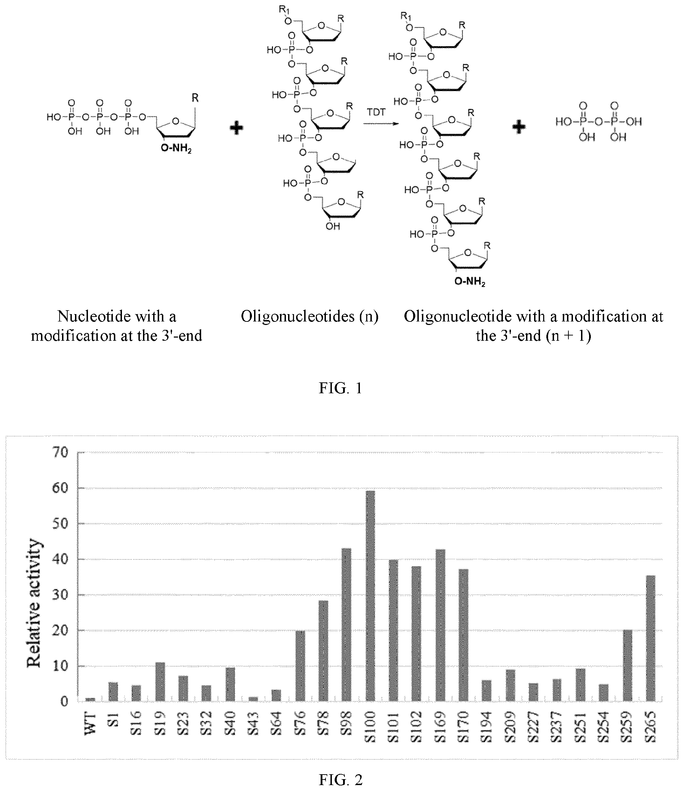

In the presence of the initiating sequence, ZaTdT catalyzed the catalytic reaction for incorporating deoxyribonucleotides with an O-amino modification group at the 3' end to the 3 end of the initiating sequence, as shown in FIG. 1.

### 2. In vitro reactions with pure enzymes

Reaction system: 100 mM NaCl, 0.25 mM CoCl₂, 50 mM KAc, 10 mM Mg(Ac)₂, pH 6.8. Substrate: 1 µM initiating strand (16 nt: ACTAGGACGACTCGAATT), 100 µM deoxyribonucleotide with an O-amino modification group at the 3' end. Enzyme: 50 µ M TdT protein and mutants.

Reaction conditions: the reaction was initiated after addition of the enzyme, reacted at 30 °C for different times, followed by inactivation of the protein at 95 °C. The mixture was centrifuged to remove the precipitate. After centrifugation, a supernatant sample was detected and analyzed using 20% urea-polyacrylamide gel (denaturing PAGE) for 3 h at 300 V. The gel was stained with 1× SYBR Gold for 20 min and the net optical density of each DNA band in the picture was further analyzed with a fluorescence gel imager from Tanon. The nucleotide incorporation efficiency was obtained by dividing the net optical density of the DNA band after nucleotide incorporation (net optical density for 17 nt) by the net optical density of the overall DNA band (net optical densities for 16 nt and 17 nt). The incorporation efficiency was multiplied by the substrate concentration and then divided by the reaction time to obtain the catalytic efficiency of the variant enzyme reaction.

The results of denaturing PAGE analysis of N-terminal amino acid deletion or addition in ZaTdT are shown in Table 2, indicating that ZaTdT and the variants of ZaTdT with N-terminal amino acid deletion or addition are able to bind to the substrate, and the deletion or addition of some amino acids at the N-terminus does not affect the catalytic efficiency of ZaTdT upon calculation.

The catalytic efficiencies of ZaTdT and some single-site or multi-site variants are shown in Table 3. The catalytic efficiency of ZaTdT and some single-site or multi-site variants was divided by that of the wild-type TdT to obtain the relative activity of TdT mutants. The relative activities of some variants are shown in FIG. 2.

The results show that the catalytic efficiency of the various variants is significantly improved compared to the wild type, with a maximum improvement of about 60-fold.

**Table 2. Catalytic efficiency of ZaTdT with deletion or addition of amino acids at N-terminus**

| Enzymes | Catalytic efficiency nM/min |
|---|---|
| ZaTdT | 4.40±0.23 |
| ZaTdT-JD | 4.35±0.18 |
| MBP-ZaTdT | 4.65±0.31 |
| MBP-ZaTdT-JD | 4.52±0.27 |

**Table 3. Catalytic efficiency of ZaTdT and some mutants**

| Enzymes | Catalytic efficiency nM/min |
|---|---|
| WT | 4.40 |
| S1 | 24.82 |
| S16 | 20.68 |
| S19 | 48.88 |
| S23 | 31.68 |
| S32 | 20.24 |
| S40 | 42.37 |
| S43 | 6.78 |
| S64 | 15.52 |
| S76 | 88.22 |
| S78 | 124.65 |
| S98 | 189.95 |
| S100 | 260.61 |
| S101 | 175.47 |
| S102 | 167.95 |
| S169 | 188.85 |
| S170 | 164.03 |
| S194 | 26.93 |
| S209 | 39.42 |
| S227 | 23.32 |
| S237 | 28.78 |
| S251 | 41.54 |
| S254 | 21.65 |
| S259 | 88.66 |
| S265 | 156.68 |

### Example 5. Use of TdT Variants in Single-Stranded Nucleotide Synthesis

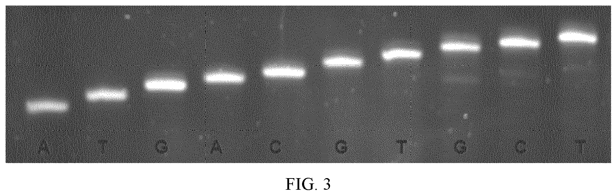

(1) Immobilization: the biotin-labeled primer was coupled to streptavidin magnetic beads at 30° C for 20 minutes.
(2) Reaction: the magnetic beads combined with the initiating strand were placed in the following reaction system: 50 mM PBS, 0.25 mM CoCl₂, 0.25 mM nucleotide with 3'-OH modified by an O-amino group, and 1.5 mg/mL TdT variant (S100). The reaction was carried out at 30 °C for 10 min.
(3) Deprotection: the reaction was deprotected with 0.7 M nitrous acid solution and then washed twice with a phosphate buffer.
(4) Steps (2) and (3) were repeated and cycled 9 times.
(5) The sample from each cycle was subjected to 20% urea polyacrylamide gel (denaturing PAGE) detection. The results after 1× SYBR Gold staining are shown in FIG. 3.

The results show that the TdT variant S100 can complete synthesis of 10 nucleotides and can be applied to single-stranded nucleotide synthesis. It can be seen that the variants contained in Table 1 or Table 3, which have the same function of incorporating modified nucleotides at the ends of single-stranded oligonucleotides as the 5100 variant, can be used in single-stranded DNA synthesis.

### Sequence information:

SEQ ID NO.1: 
MDRFKAPAVISQRKRQKGLHSPKLSCSYEIKFSNFVIFIMQRKMGLTRRMFLMELGRRKGF
RVESELSDSVTHIVAENNSYLEVLDWLKGQAVGDSSRFELLDISWFTACMEAGRPVDSEV
KYRLMEQSQSLPLNMPALEMPAFIATKVSQYSCQRKTTLNNYNKKFTDAFEVMAENYEFK
ENEIFCLEFLRAASLLKSLPFSVTRMKDIQGLPCVGDQVRDIIEEIIEEGESSRVNEVLNDERY
KAFKQFTSVFGVGVKTSEKWYRMGLRTVEEVKADKTLKLSKMQKAGLLYYEDLVSCVSK
AEADAVSLIVKNTVCTFLPDALVTITGGFRRGKNIGHDIDFLITNPGPREDDELLHKVIDLW
KKQGLLLYCDIIESTFVKEQLPSRKVDAMDHFQKCFAILKLYQPRVDNSTCNTSEQLEMAE
VKDWKAIRVDLVITPFEQYPYALLGWTGSRQFGRDLRRYAAHERKMILDNHGLYDRRKRI
FLKAGSEEEIFAHLGLDYVEPWERNA
SEQ ID NO.2: 
LPLNMPALEMPAFIATKVSQYSCQRKTTLNNYNKKFTDAFEVMAENYEFKENEIFCLEFLR
AASLLKSLPFSVTRMKDIQGLPCVGDQVRDIIEEIIEEGESSRVNEVLNDERYKAFKQFTSVF
GVGVKTSEKWYRMGLRTVEEVKADKTLKLSKMQKAGLLYYEDLVSCVSKAEADAVSLIV
 
KNTVCTFLPDALVTITGGFRRGKNIGHDIDFLITNPGPREDDELLHKVIDLWKKQGLLLYCD
IIESTFVKEQLPSRKVDAMDHFQKCFAILKLYQPRVDNSTCNTSEQLEMAEVKDWKAIRVD
LVITPFEQYPYALLGWTGSRQFGRDLRRYAAHERKMILDNHGLYDRRKRIFLKAGSEEEIF
AHLGLDYVEPWERNA
SEQ ID NO.3: 
MKIEEGKLVIWINGDKGYNGLAEVGKKFEKDTGIKVTVEHPDKLEEKFPQVAATGDGPDII
FWAHDRFGGYAQSGLLAEITPDKAFQDKLYPFTWDAVRYNGKLIAYPIAVEALSLIYNKDL
LPNPPKTWEEIPALDKELKAKGKSALMFNLQEPYFTWPLIAADGGYAFKYENGKYDIKDV
GVDNAGAKAGLTFLVDLIKNKHMNADTDYSIAEAAFNKGETAMTINGPWAWSNIDTSKV
NYGVTVLPTFKGQPSKPFVGVLSAGINAASPNKELAKEFLENYLLTDEGLEAVNKDKPLGA
VALKSYEEELVKDPRIAATMENAQKGEIMPNIPQMSAFWYAVRTAVINAASGRQTVDEAL
KDAQTNSSSNNNNNNNNNNLGMDRFKAPAVISQRKRQKGLHSPKLSCSYEIKFSNFVIFIM
QRKMGLTRRMFLMELGRRKGFRVESELSDSVTHIVAENNSYLEVLDWLKGQAVGDSSRFE
LLDISWFTACMEAGRPVDSEVKYRLMEQSQSLPLNMPALEMPAFIATKVSQYSCQRKTTL
NNYNKKFTDAFEVMAENYEFKENEIFCLEFLRAASLLKSLPFSVTRMKDIQGLPCVGDQVR
DIIEEIIEEGESSRVNEVLNDERYKAFKQFTSVFGVGVKTSEKWYRMGLRTVEEVKADKTL
KLSKMQKAGLLYYEDLVSCVSKAEADAVSLIVKNTVCTFLPDALVTITGGFRRGKNIGHDI
DFLITNPGPREDDELLHKVIDLWKKQGLLLYCDIIESTFVKEQLPSRKVDAMDHFQKCFAIL
KLYQPRVDNSTCNTSEQLEMAEVKDWKAIRVDLVITPFEQYPYALLGWTGSRQFGRDLRR
YAAHERKMILDNHGLYDRRKRIFLKAGSEEEIFAHLGLDYVEPWERNA
SEQ ID NO.4:

MKIEEGKLVIWINGDKGYNGLAEVGKKFEKDTGIKVTVEHPDKLEEKFPQVAATGDGPDII
FWAHDRFGGYAQSGLLAEITPDKAFQDKLYPFTWDAVRYNGKLIAYPIAVEALSLIYNKDL
LPNPPKTWEEIPALDKELKAKGKSALMFNLQEPYFTWPLIAADGGYAFKYENGKYDIKDV
GVDNAGAKAGLTFLVDLIKNKHMNADTDYSIAEAAFNKGETAMTINGPWAWSNIDTSKV
NYGVTVLPTFKGQPSKPFVGVLSAGINAASPNKELAKEFLENYLLTDEGLEAVNKDKPLGA
VALKSYEEELVKDPRIAATMENAQKGEIMPNIPQMSAFWYAVRTAVINAASGRQTVDEAL
KDAQTNSSSNNNNNNNNNNLGLPLNMPALEMPAFIATKVSQYSCQRKTTLNNYNKKFTD
AFEVMAENYEFKENEIFCLEFLRAASLLKSLPFSVTRMKDIQGLPCVGDQVRDIIEEIIEEGES
SRVNEVLNDERYKAFKQFTSVFGVGVKTSEKWYRMGLRTVEEVKADKTLKLSKMQKAG
LLYYEDLVSCVSKAEADAVSLIVKNTVCTFLPDALVTITGGFRRGKNIGHDIDFLITNPGPRE
DDELLHKVIDLWKKQGLLLYCDIIESTFVKEQLPSRKVDAMDHFQKCFAILKLYQPRVDNS
TCNTSEQLEMAEVKDWKAIRVDLVITPFEQYPYALLGWTGSRQFGRDLRRYAAHERKMIL
DNHGLYDRRKRIFLKAGSEEEIFAHLGLDYVEPWERNA

The embodiments of the present disclosure have been described above. However, the present disclosure is not limited to the embodiments described above. Any modification, equivalent, improvement and the like made without departing from the spirit and principle of the present disclosure shall fall within the protection scope of the present disclosure.

## Claims

1. A variant of terminal deoxynucleotidyl transferase (TdT),
wherein the TdT variant is any one or a combination of site mutants or terminal variants of ZaTdT SEQ ID NO.1 derived from *Zonotrichia albicollis*:
the site mutant being selected from any one or a combination of the following, wherein the mutation position indicated is determined by alignment with SEQ ID NO.1:
i) an amino acid sequence of the variant comprising at least one mutation of a residue in at least one position selected from Y178, F186, I210, I228, V302, D324, R353, D324, T330, G331, G332, F333, R335, K337, I329, I339, G340, H341, I343, R435, G452, W453, T454, G455, S456, R457, N477, and H478, or at least one mutation of the functionally equivalent residue;
ii) preferably, the amino acid sequence of the variant comprising at least one mutation of a residue in at least one position selected from Y178, F186, I210, I228, V302, T330, R335, K337, I339, G340, R435, T454, G455, S456, R457, N477, and H478, or at least one mutation of the functionally equivalent residue;
iii) more preferably, the amino acid sequence of the variant comprising at least one mutation of a residue in at least one position selected from Y178A, F186R, I210L, I228L, V302G, T330A, R335A, R335L, K337A, K337G, K337L, K337V, I339V, G340V, G340L, R435L, T454A, G455V, S456V, R457G, N477L, and H478G, or at least one mutation of the functionally equivalent residue;
iv) preferably, the amino acid sequence of the mutant comprising mutations of amino acid residues at any two sites of Y178, F186, I210, I228, V302, D324, R353, D324, T330, G331, G332, F333, R335, K337, I329, I339, G340, H341, I343, R435, G452, W453, T454, G455, S456, R457, N477, and H478;
v) preferably, the amino acid sequence of the variant comprising mutations of amino acid residues at any two sites of Y178, F186, I210, I228, V302, T330, R335, K337, I339, G340, R435, T454, G455, S456, R457, N477, and H478;
vi) more preferably, the amino acid sequence of the mutant comprising mutations of amino acid residues at two sites of Y178, F186, I210, I228, V302, T330, R335, K337, I339, G340, R435, T454, G455, S456, R457, N477, and H478;
vii) preferably, the amino acid sequence of the mutant comprising mutations at the following two sites: Y178/F186, I210/I228, R335/K337, I339/G340, and S456/R457;
viii) preferably, the amino acid sequence of the mutant comprising mutations at the following two sites: Y178A/F186R, I210L/I228L, R335A/K337A, R335L/K337G, R335L/K337L, R335L/K337V, I339V/G340V, and S456V/R457L; and
the terminal variant being selected from any one or a combination of the following:
i) a variant with addition or deletion of some amino acids at the N-terminus and/or C-terminus of SEQ ID NO.1 or a variant thereof; preferably, a sequence of the variant being set forth in SEQ ID NO.2; and
ii) a variant with inclusion of a tag sequence, such as MBP tag, HIS tag, GST tag or the like, at the N-terminus and/or C-terminus of SEQ ID NO.1 or a variant thereof; preferably, a sequence of the variant being set forth in SEQ ID NO.3 or SEQ ID NO.4;
wherein the TdT variant is capable of incorporating modified nucleotides, preferably 3'-O-modified nucleotides, and more preferably 3'-O-blocked nucleotides in a nucleic acid fragment; and
the TdT variant has at least 80% identity to SEQ ID NO.1 or a functionally equivalent sequence, more preferably at least 85%, 90%, 95%, 96%, 97%, 98%, or 99% identity to SEQ ID NO.1 or a functionally equivalent sequence.

2. The variant as claimed in claim 1, wherein the variant further comprises at least one mutation of residues in at least a semi-conserved region of X1X2X3X4RX5GX6NX7X8X9DX10 (SEQ ID NO. 1) from position 330 to position 343 of the sequence, wherein
X1 represents a residue selected from A, I, L, V, P, Q, T, K, and E,
X2 represents a residue selected from G, L, V, F, Y, C, H, R, and D,
X3 represents a residue selected from A, I, L, V, P, Q, T, K, and E,
X4 represents a residue selected from G, L, V, F, Y, C, H, R, and D,
X5 represents a residue selected from A, I, L, V, P, Q, T, K, and E,
X6 represents a residue selected from G, L, V, F, Y, C, H, R, and D,
X7 represents a residue selected from A, I, L, V, P, Q, T, K, and E,
X8 represents a residue selected from G, L, V, F, Y, C, H, R, and D,
X9 represents a residue selected from A, I, L, V, P, Q, T, K, and E, and
X10 represents a residue selected from G, L, V, F, Y, C, H, R, and D.

3. The variant as claimed in claim 1 or 2, wherein the variant further comprises at least one mutation of residues in at least the semi-conserved region of ALLX1X2X3X4X5X6QFG (SEQ ID NO. 1) from position 449 to position 460 of the sequence, wherein
X1 represents a residue selected from A, I, L, V, P, Q, T, K, and E,
X2 represents a residue selected from G, L, V, F, Y, C, H, R, and D,
X3 represents a residue selected from A, I, L, V, P, Q, T, K, and E,
X4 represents a residue selected from G, L, V, F, Y, C, H, R, and D,
X5 represents a residue selected from A, I, L, V, P, Q, T, K, and E, and
X6 represents a residue selected from G, L, V, F, Y, C, H, R, and D.

4. The variant of the terminal deoxynucleotidyl transferase (TdT) as claimed in any one of claims 1 to 3, wherein the variant comprises at least one combination of substitutions selected from combinations of substitutions disclosed in Table 1, or functionally equivalent residues; preferably, the variant comprises at least one combination of substitutions selected from combinations of substitutions disclosed in Table 3, or functionally equivalent residues.

5. A nucleic acid molecule encoding the variant as claimed in any one of claims 1 to 4.

6. An expression vector comprising the nucleic acid molecule as claimed in claim 5.

7. A host cell comprising the nucleic acid molecule as claimed in claim 5 or the expression vector as claimed in claim 6.

8. A Method for producing the variant as claimed in any one of claims 1 to 4, wherein the host cell as claimed in claim 7 is cultured under a culture condition allowing the expression of the nucleic acid encoding the variant, and wherein the variant is optionally recovered.

9. Use of the variant as claimed in any one of claims 1 to 4 for the synthesis of a nucleic acid molecule with 3'-O-modified nucleotides in the absence of a template.

10. A method for synthesizing a nucleic acid molecule in the absence of a template, comprising a step of contacting a nucleic acid primer with both at least one nucleotide, preferably at least one 3'-O-modified nucleotide, and the variant as claimed in any one of claims 1 to 4.

11. A kit, enzyme or composition for performing a nucleotide incorporation reaction, comprising a variant of TdT as defined in any one of claims 1 to 4;
preferably, further comprising one or more nucleotides or nucleic acid molecules; more preferably, further comprising one or more 3'-O-modified nucleotides; and
still more preferably, further optionally comprising at least one DNA template or nucleic acid primer.
